Europäisches Patentamt

European Patent Office

Office européen des brevets

(11) Publication number: **0 503 454 A1**

## EUROPEAN PATENT APPLICATION

(21) Application number: **92103667.9**

(22) Date of filing: **04.03.92**

(51) Int. Cl.5: **G01N 33/53, G01N 33/546**

(30) Priority: **15.03.91 US 669783**

(43) Date of publication of application:
**16.09.92 Bulletin 92/38**

(84) Designated Contracting States:
**BE CH DE ES FR GB IT LI NL**

(71) Applicant: **F. HOFFMANN-LA ROCHE AG**
**Postfach 3255**
**CH-4002 Basel(CH)**

(72) Inventor: **Antonian, Edna**
**186 Watchung Drive**
**Hawthorne, N.J. 07506(US)**

Inventor: **Courtney, Jodi Blake**
**407 Valley Road**
**Upper Montclair, N.J. 07043(US)**
Inventor: **Passarelli, Joseph John**
**34 Everitts Road**
**Ringoes, N.J. 08551(US)**
Inventor: **Salamone, Salvatore Joseph**
**37 E. Pierrepont Avenue**
**Rutherford, N.J. 07070(US)**

(74) Representative: **Notegen, Eric-André et al**
**Grenzacherstrasse 124 Postfach 3255**
**CH-4002 Basel(CH)**

(54) Agglutination immunoassay.

(57) A method for enhancing the reactivity of microparticle based immunoassays has been developed using polyvinylpyrrolidones (PVP). Microparticle aggregation reactions are accelerated resulting in increased standard curve dynamics and improved sensitivity.

FIGURE - 1

EP 0 503 454 A1

The present invention relates to the use of PVP to improve microparticle based agglutination immunoassays and significantly enhance the resulting sensitivity and signal of these immunoassays.

More particularly, the present invention provides an improvement in a microparticle based immunoassay method for the detection of target molecules in a sample of body fluid by agglutinometric determination, the improvement comprising adding to said sample an amount of polyvinylpyrrolidone (PVP) effective to increase the sensitivity and signal of said immunoassay.

The use of microparticles in diagnostic assays is now generally well known. The assay method may be used in both homogeneous and heterogeneous formats, for both large molecules (e.g., proteins, hormones, enzymes, antibodies, immunocomplexes) and small (e.g. therapeutic drugs, abused drugs and their metabolites), in competitive and direct binding assays. A variety of polymeric microparticle systems have been developed which allow the covalent or non-covalent attachment of compounds of biological interest (see, for example, those discussed in U.S. Patent No. 4,480,042 at Column 2). Widely used are particles, of a variety of sizes, made of monomers co-polymerized with styrene.

The various embodiments of microparticle technology in the immunoassay have in common a solid support which is the carrier for one component of an immunological binding pair. The immunochemical reaction may affect a change in the state of dispersion of the particles or a second label could be used when the particles serve only to isolate a determinant on a solid support. Where the particles are specifically cross-linked (or inhibited therefrom) as a result of the interaction of the binding pair, there are a variety of analytical methods that may be used for signal detection, such as turbidity, nephelometry, angular anisotropy, quasi-elastic light scattering and so forth. Not only are there a variety of such agglutinometric techniques available, but the method is also applicable to several instruments of varying degrees of automation.

Initial application of microparticles in diagnostics was for the detection of serologically determinant factors in body fluids (Howanitz J. H., Immunoassay-Innovations in Label Technology. Arch Pathol Lab Med, Vol 112, pp. 775-779, (1988); Hechemy K. E., and Michaelson E. E., Latex Particle Assay in Laboratory Medicine. Part I, Laboratory Management (June 1984). An agglutinator selective for the antigen of interest could be bound (covalently or absorbed) on the particle surface. Cross-linking and detection are optimized where the antigen has several determinants available for agglutinator binding. Where there is no antigen present in a sample, no change in the state of the particle dispersion would be detected. The approach is not optimal for the detection of low molecular weight haptens. However, when a bivalent agglutinator is the soluble binding component the analyte competes with the hapten on the carrier for agglutinator binding sites. Thus, the presence of hapten in the sample inhibits particle cross-linking. The improvement in sensitivity thus obtained, has increased the utility of microparticle assays in diagnostic testing for both haptens, such as abused and therapeutic drugs, and antigens such as human chorionic gonadotropin (U.S. Patent 3,857,931). While these assays depend on the detection of flocculation, the sensitivity obtained with a soluble agglutinator sacrifices signal where the agglutinator is not in excess over the range of clinical significance.

The use of polyethylene glycol (PEG) and various detergents as agglutination enhancers has been suggested. U.S. Patent No. 4,480,042 at column 10; Galloway, R.J., Development of Microparticle Tests and Immunoassays page 29 (Seradyn, Inc. Indianapolis, 1988). The stabilization of dispersions of microparticles, as well as inhibition of "non-specific agglutination", has been ascribed to surfactants and water soluble polymers including polyvinylpyrrolidone (PVP). Bangs, L. B., Uniform Latex Particles, pp. 25-26 and 55-56 (Seradyn Inc., 1984). PVP-360 in low concentrations, i.e., to a final concentration below 0.4%, is incorporated in the latex microparticle reagents used in the capillary mediated agglutinographic slides commercially sold as ONTRAK™ (Roche Diagnostic Systems, Inc.) for drugs of abuse testing. (The final concentrations referred to in the specification and claims herein are referenced to fully made up reaction mixtures including all reagents plus sample; all percentages are weight/volume unless otherwise noted.)

Brief Description of the Drawings

Figure 1 graphically demonstrates data generated as in Example 1, a THC calibration curve.

Figure 2 represents the optimization of a THC assay by use of PVP 360 at various concentrations in accordance with Example 2.

Figure 3 demonstrates optimization of a calibration curve for Benzodiazepine assay by use of PVP-360 and PVP-40, in accordance with Example 3, versus no addition of PVP-40.

Figure 4 demonstrates enhancement of the competitive microparticle aggregation reaction for Benzodiazepine by use of PVP-40 at various concentrations, in accordance with Example 4.

In accordance with the present invention, it was unexpectedly been found that the addition of effective amounts of PVP to microparticle based immunoassays results in enhanced reaction kinetics thereby yielding signals of increased accuracy and an assay of improved sensitivity and overall signal to noise ratio. When used in sufficient concentration the PVP works as a reaction enhancer, rather than merely as a stabilizer of the dispersion. PVP of any molecular weight can be utilized in the present invention.

The microparticle reagents utilized in the present invention incorporate microparticles that are coated with one component of an immunological binding pair which binding pair is diagnostically selective for the substance of interest. The microparticles remain monodispersed. Upon kinetic interaction in the presence of the complementary binding partner the microparticles aggregate resulting in changes of optical density. Enhanced reaction kinetics yield signals of increased accuracy and an assay of improved sensitivity. Significant slopes are obtained for the calibration curves so as to make quantitation possible.

The present invention provides enhancement of specific analyte-agglutinator binding mediated by a particle carrier so as to permit the design of diagnostc assays with sufficient clinical sensitivity for application to the detection of haptens and antigens. Specific representative analytes in human body fluid include, for example, $\Delta^9$-carboxytetrahydrocannabinol (COOH-THC), diazepam, human chorionic gonadotropin, and human gamma globulin.

In general, increasing concentrations of PVP yield increased sensitivity and signal. Moreover, as noted previously the same effect is achieved by increasing the molecular weight of the PVP. PVP is typically available commercially in different grades sized according to average molecular weight, e.g., PVP-40 (avg. M.W. = 40 kD), PVP-360 (avg. M.W. = 360 kD), etc. These standard commercial grades may consist of a fairly broad distribution of molecular weights of the polymer. In accordance with the present invention it has been found that the lower molecular weight grades of PVP (e.g., PVP-40) are effective when added to a final concentration from about 3%, whereas the higher weights (e.g., PVP-360) can be effective in amounts from 0.6%. Blending the weights tends to cause a corresponding averaging of the beneficial effects of the different weights, and in some instances may even yield a synergistic-like enhancement, allowing for the advantageous use of lesser amounts of a given PVP in combination with other PVP's.

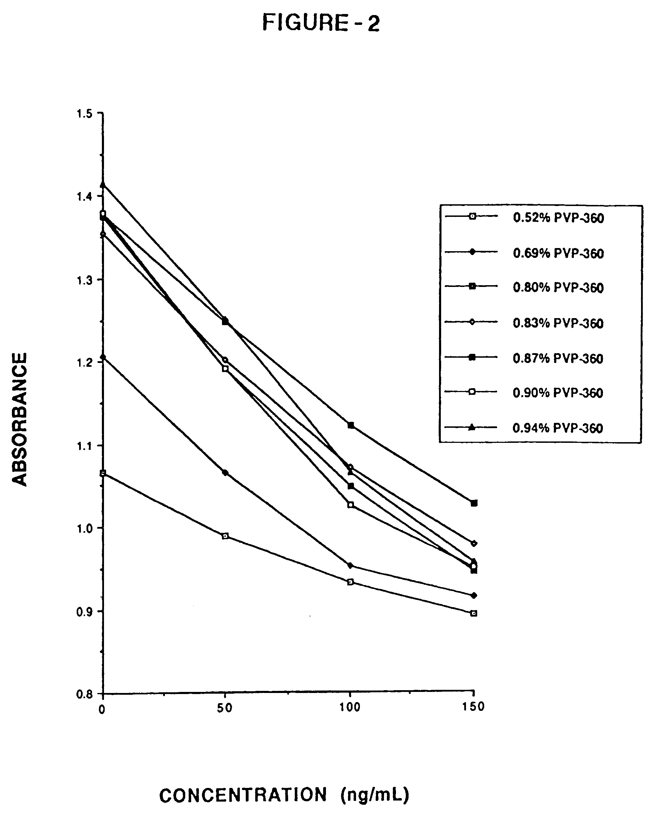

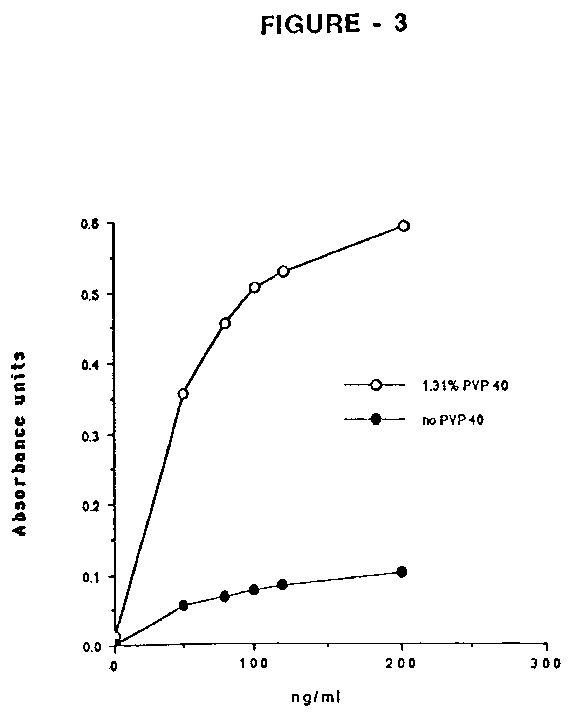

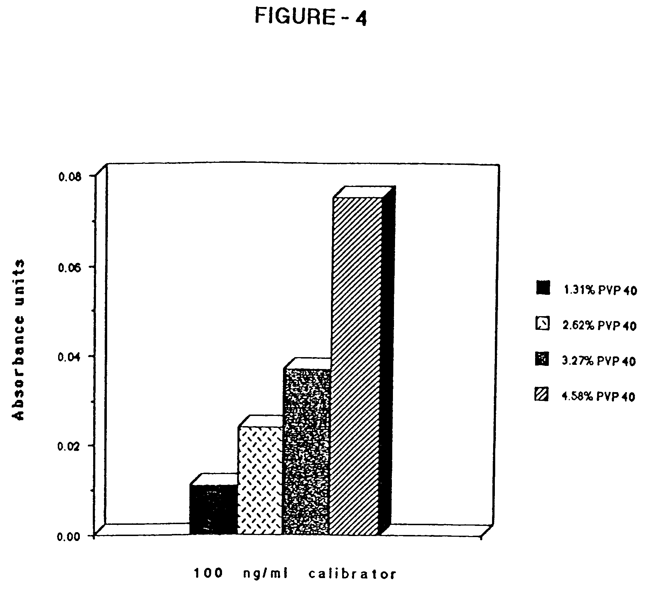

Thus, PVP-360 gives greater acceleration of the reaction than does PVP-40 at the same concentrations. In an assay for THC, for example, PVP-360 works well at final concentration of 0.69% (see Figure 2). After a concentration of 1.50% the amount of increase in acceleration levels off. In the case of PVP-40, the 3.27% concentration in the final reaction mixture more than triples the absorbance signal from that for PVP at 1.31%, (See Figure 4). The same 3.27% concentration of PVP-40, however, barely matches the enhancement that addition of only 0.77% PVP-360 provides (Compare Figure 3).

In a preferred embodiment of the present invention the PVP used has an average molecular weight of about 360 kD and is added to a final concentration of at least about 0.6%.

In certain cases it may be advantageous to use high and low molecular weights together. For example, in a benzodiazepine assay reagent prepared for use on the Olympus analyzer (Example 3), the instrument could not accurately pipet the PVP containing reagent if the concentration of PVP-360 in the antibody & diluent reagent reservior (R1) was above 1.18%. The addition of PVP-40 to a concentration of 2.0% in R1 (= 1.31 % final concentration) and PVP-360 to a concentration of 1.175% in R1 (= 0.77% final concentration) gave acceptable kinetics and good pipeting precision. (Figure 3).

The gross amount of enhancement of signal and sensitivity will, of course, reflect the inherent characteristics of each assay and intrinsic factors such as, for example, antibody affinity, as well as extrinsic factors such as apparatus limitations. However, as can be appreciated from optimization graphs such as depicted in Figures 2 and 4, the skilled worker can readily titrate to a selection of PVP molecular weight grade and concentration, or combination thereof, which are optimal for any particular assay system or apparatus.

The upper limit of concentration is that which begins to impair the agglutination reaction or presents some other practical difficulty in the operation of the assay, usually because of excessive viscosity effects, and in part depending on the apparatus used.

The improved immunoassay reagents and method of the present invention may be usefully employed in any of the immunoassay formats susceptible to an instrumental method for the measurement of the changes brought about by the agglutination reaction. Both manual as well as automated apparatus testing may be suitably employed for such agglutinometric analysis. Typically, automated instrumentation will operate utilizing a multiplicity of reagent containers or reservoirs from which will be pipetted the appropriate amount of each reagent for addition to the sample. For immunoassays such as the subject agglutination assays, this will usually involve at least two such containers; typically, one for an antibody reagent and the other for the microparticles bound with the corresponding antigenic determinant(s). Additional containers/reservoirs may be present in some instruments containing diluent, buffers and/or other additives for appropriate treatment of the sample. Of course, the present invention may be conveniently effected by incorporating appropriate amounts of PVP in one or more of these containers so that the effective final concentration in the sample is achieved. Therefore, it is an object of the present invention to provide a diagnostic kit for a microparticle based immunoassay for the detection of target molecules in a sample of body fluid by agglutinometric determination comprising a first container containing a reagent comprising antibody selective for said target molecules and a second container containing a reagent comprising microparticles to which are bound immunologically active haptens or antigens selective for said target molecules, the improvement comprising including in either container or the combination of the two an amount of PVP effective to increase the sensitivity and signal of said immunoassay. Being aware of the proviso that excessive amounts of PVP may be detrimental to the microparticle containing reagents it may be preferable to put the PVP in the complementary immunoreagent without microparticle. Under this circumstances the present invention provides a reagent for a microparticle based immunoassay comprising antibodies selective for the target molecules and an amount of PVP effective to increase the sensitivity and signal of said immunoassay. Preferably the PVP will be in the diluent/buffer reagent if such a reagent will be used as an additional part. In this case a diagnostic kit for a microparticle based immunoassay for the detection of target molecules in a sample of body fluid by agglutinometric determination comprising a first container containing a reagent comprising antibody selective for said target molecules, a second container containing a reagent comprising microparticles to which are bound immunologically activated hapten selective for said target molecules, and a third container containing a reagent comprising an aqueous diluent, the improvement comprising including in one of said containers or a combination thereof an amount of PVP effective to increase the sensitivity and signal of said immunoassay, may be applied. Preferably the PVP used in the reagents or diagnostic kits has an average molecular weight of about 360 kD and is present in an amount sufficient to provide a final concentration of at least about 0.6% in said immunoassay.

The following examples represent illustrative embodiments of the invention:

Example 1

Tetrahydrocannabinoid Assay

Preparation of trans-rac-1-[[(6a,7,10,10a-Tetrahydro-1-hydroxy-6,6-dimethyl-3-pentyl-6H-dibenzo[b,d]pyran-9-yl(carbonyl)oxy]-2,5-pyrrolidinedione:

To a solution of 1.26g (3.67 m mole) of 11-nor-$\Delta^8$-THC-9-carboxylic acid[1] in 50 mL of 1:1 ethylacetate/methylene chloride is added 1.68 g (14.7 mmole) of N-hydroxy-succinimide and 2.10 g (11.0 mmole) of 1-(3-dimethyl-aminopropyl)3-ethylcarbodiimide hydrochloride. The reaction is stirred for 16 hours at room temperature. The reaction mixture is washed with 1N hydrochloric acid (3 x 100 mL), water (3 x 100 mL) and saturated brine (100 mL). The organic phase is dried over $Na_2SO_4$ and concentrated under reduced pressure. Chromatography over silica gel (Flash, 2 inches x 6 inches). Eluting with 40% (v/v) ethylacetate in hexane afforded 1.41g (87%) of the desired N-hydroxysuccinimide ester. NMR, IR and MS consistent with structure were obtained.

Conjugation of trans-rac-1-[[(6a, 7, 10, 10a-Tetrahydro-1-hydroxy-6,6-dimethyl-3-pentyl-6H-dibenzo[b,d]pyran-9-yl)carbonyl]oxy]-2,5-pyrrolidinedione (cannabinoid) to Bovine Serum Albumin (BSA):

References:

I. J. Org. Chem. 51, pp. 5463-5465 (1986).

Materials:

Bovine Serum Albumin (BSA), Fraction V, Reagent Grade, cannabinoid derivative, Molecular Weight 441.52 mg/mM, Dimethyl Sulfoxide (DMSO), 50 mM postassium phosphate buffer pH = 7.5 (KPi buffer), Stock solution of cannabinoid (5 mg/mL): 10 mg of cannabinoid derivative in 2.0 mLs of DMSO, Dialysis membrane 25,000 MWCO.

Method:

The BSA to be conjugated is prepared by dissolving 125 mg in 1.25 mL of KPi buffer (100 mg BSA/mL buffer). Once the protein is dissolved, the solution is cooled in an icebath. While mixing on a magnetic stir plate DMSO (1.875 mL) is added dropwise to the protein solution which is then allowed to come to room temperature. At this point the stock solution of cannabinoid may be freshly prepared (see above). The stock solution (0.167 mL) of cannabinoid is added dropwise to the protein in DMSO/buffer. The reaction continues to stir overnight at room temperature. Excess DMSO and unbound hapten are removed in an extensive (greater than ten million fold total) dialysis step. The conjugate is initially dialyzed at room temperature against 15 mL DMSO diluted with 35 mL of KPi buffer and then against 5 mL of DMSO diluted with 45 mL of KPi buffer. Dialysis then continues at 4°C against KPi buffer. Conjugate recovered is evaluated for protein concentration at 280nm.

Preparation of sensitized microparticle containing the cannabinoid-BSA conjugate:

Materials:

Carboxylated polystyrene microparticles (diameter 0.1 to 0.13 um manufactured by Seradyn, Inc.), N,N-Dimethylformamide (DMF), 1-Hydroxybenzotriazole (NHB;$H_2O$), 1-Cyclohexyl-3-(2-Morpholinoethyl) carbodiimide metho-p-toluene sulfonate (CMC), Triton X-100, cannabinoid-BSA conjugate, 50 mM Sodium Bicarbonate pH 8.6, BSA Fraction V Reagent Grade, buffer of 10 mM KPi, pH 7.5, 0.1% sodium azide, and 0.1% triton X-100 (microparticle storage buffer).

Methods:

The carboxylated microparticles (2.0 mL of 10% solids), as supplied by the manufacturer is washed to exchange the detergent. Triton X-100 at 0.1% in deionized water is used for a total dilution of greater than 1:1,000,000 by volume. The washed latex in 0.1% Triton X-100 is adjusted to 3% solids in 0.1% Triton X-100 from a standard curve of latex concentration at 500 nm.

A stock solution of NHB is prepared by dissolving 110 mg NHB in 2.2 mL of DMF to which deionized water (2.2 mL) is added (25 mg NHB/mL). While rapidly stirring the prepared microparticles solution (6.7 mL) at room temperature the NHB solution (0.42 mL) is added rapidly dropwise. The solution is stirred for 10 minutes during which the stock solution of CMC is made up.

The stock solution of CMC is prepared by dissolving 300 mg CMC in 6.0 mL (50 mg CMC/mL) of deionized water. With rapid stirring 0.581 mL are added rapidly dropwise to the microparticle solution prepared above. Following this addition the reaction is stirred for three hours at room temperature. The excess activating reagents are removed by again washing the microparticle preparation with 0.1% Triton X-100 for a total dilution of 1:1,000,000. The microparticles are adjusted to 2% solids by comparison to a standard curve of microparticle concentration at 500nm.

The BSA conjugate prepared previously is used in the following way to sensitize the microparticles. The conjugate (12.5 mg) is diluted to 5 mg conjugate BSA/mL with 50 mM sodium bicarbonate pH 8.5. Bovine Serum Albumin Fraction V, reagent grade (37.5 mg) is dissolved in 7.5 mL of sodium bicarbonate (5 mg BSA/mL) pH 8.5. The BSA (7.5 mL) and BSA conjugate (2.5 mL) solutions are then combined for 10mL total volume at 5 mg BSA/mL. While vigorously mixing the protein solution, the activated microparticles (10 mL) are added rapidly. The reaction is mixed overnight. Unbound BSA conjugate is then removed by extensive washing. The final latex suspension is diluted with 10 mM KPi, 0.1% Triton-X-100, 0.1% sodium azide, pH 7.5 and 10 mM KPi, 0.1% sodium azide, pH 7.5 for a final microparticle reagent at 0.7% solids in 10 mM KPi, 0.07% Triton-X-100, 0.1% sodium azide, pH 7.5. The percent solids of the microparticles is determined by comparison to a standard curve of microparticle concentration at 500 nm.

Preparation of Antisera reagent for test:

Mouse Monoclonal antibody selective against cannabinoids is diluted appropriately in an aqueous solution at pH 6.5:
1. 0.05M HEPES [4-(2-hydroxymethyl)-1-piperazineethanesulfonic acid and sodium salt]
2. 0.01% Bovine Serum Albumin
3. 0.5% Sodium Chloride
4. 0.1% Sodium Azide

Preparation of Sample Diluent for test:

The reaction buffer is an aqueous solution at pH 7.0
1. 0.05M PIPES [1,4-piperazinebis-(ethanesulfonic acid) and disodium salt.]
2. 2.5% PVP [polyvinylpyrrolidone] 360
3. 2.0% Sodium Chloride
4. 0.1% Sodium Azide
5. 0.025% Foamaster, FLD

Assay for cannabinoid abuse:

The diagnostic screening assay is performed on the ROCHE COBAS MIRA. Standards are prepared by the addition of $\Delta^9$- COOH THC to drug free normal human urine containing 0.1% sodium azide. The clinical analyzer pipettes the onboard reagents and samples into one cuvette where the competitive agglomeration reaction occurs and measurement of the turbidity is made. Reagent transfer is accomplished in two stages. Stage one: ten microliters of urine sample are pipetted with 85uL of sample diluent into the cuvette, followed immediately by 100uL of the antibody reagent and mixing. The initial spectrophotometric reading is taken. Twenty-five seconds later stage 2: 30 uL of microparticle reagent with 20uL of water are transferred into the cuvette and the reaction is mixed. About 100 seconds after stage 2, a final measurement of the turbidity is made. The overall change in turbidity in the reaction is compared to a calibration curve, and results reported in ng/mL.

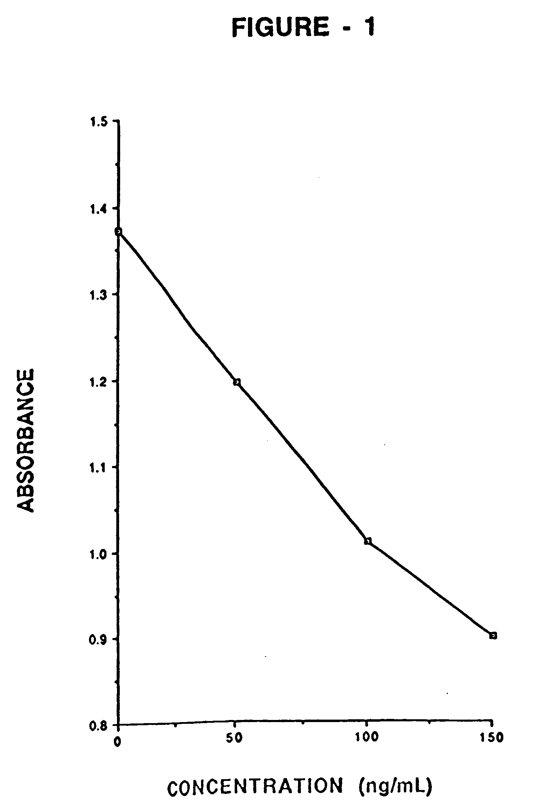

The assay components, particularly antibody titer and PVP 360 concentration have been optimized to give a standard curve with desired performance characteristics around the NIDA cut-off, X, 100ng THC/mL. See Figure 1. The assay has a low clinical background, is selective for cannabinoids, has clinically significant sensitivity [pickup], and precision to provide a >95% confidence limit to distinguish 0.8X and 1.2X from X.

Example 2

THC Assay Effect of PVP 360 Concentration

The same material and methods are used as in Example 1 except that seven Sample Diluents are prepared containing the following concentrations of PVP 360: 1.5%; 2.0%; 2.3%; 2.4%; 2.5%; 2.6% and 2.7%. The results showing the effect of the different final concentrations of PVP 360 are shown in Figure 2.

Example 3

Benzodiazepine Assay

5-((2,5-dioxo-1-pyrrolidinyl)oxy)-5-oxo-pentanoyl chloride (2)

Glutaric anhydride is reacted with one equivalent N-hydroxysuccinimide in refluxing tetrahydrofuran to give glutaric acid monosuccinimidyl ester. This is then treated with excess thionyl chloride at 45°C to afford 2.

3-Amino-7-chloro-1,3-dihydro-1-methyl-5-phenyl-2H-1,4-benzodia zepine-2-one (1)

3,7-dichloro-1-methyl-5-phenyl-1,4-benzodiazepine-2-one[1] is converted to 7-chloro-3-azidol-1-methyl-5-phenyl-1,4-benzodiazepine-2-one according to the procedure of T. Yusumitsu et al[2]. This is further converted to compound 1 according to the procedure of H. Staudinger and E. Hauser[3].

N-(7-chloro-2,3-dihydro-1-methyl-2-oxo-5-phenyl-1,4-benzodia zepine-3-yl)-5-oxo-5-((2,5-dioxo-1-pyrrolilidinyl)oxy)pentanamide (3)

To a solution mixture of 3-aminodiazepam,1-(0.5, 1.48mmole) in anhydrous THF (30mL) and dry Et$_3$N(0.5mL) at 0°C 0.46 g., (1.85 mmole) of 2 is added. After stirring for 15 minutes at 0°C, the reaction mixture is concentrated under reduced pressure, the residue dissolved in CH$_2$Cl$_2$ and

washed with H$_2$O, saturated NaHCO$_3$ and H$_2$O. The organic layer is dried over Na$_2$SO$_4$ and concentrated under reduced pressure to yield a white solid which is crystallized in Et$_2$O to yield compound 3 (595 mg. 78%, NMR, IR and MS consistent with structure).

References

1. J.Med. Chem. 22(9), 1093-1096, (1979)
2. Chem.Pharm.Bull 26(9), 2874-2879, (1978)
3. Helv.Chim.Acta. 4, 861, (1921)

Preparation of Benzodiazepine-BSA Conjugate

Materials

Bovine Serum Albumin (BSA), Fraction V, Reagent Grade Benzodiazepine-NHS derivative, DMSO (dimethyl sulfoxide), 50mM Potassium Phosphate buffer pH 7.6, Benzodiazepine stock solution (5mg/mL of DMSO), Dialysis membrane 25,000 MWCO.

Method

Eighty-nine mg/mL of BSA is dissolved in phosphate buffer of pH 7.6. To reach the final concentration of 55% DMSO in this solution, DMSO is added dropwise to the BSA/buffer solution in an ice bath while stirring. A Ten mL solution is then brought to room termperature and 1.5 mg of BDZ-NHS derivative stock is added. This is stirred overnight at room temperature. The solution is dialyzed to eliminate excess DMSO and hapten. This is done in stepwise gradient of buffers with decreasing DMSO concentration (DMSO: buffer ratios of 3:7 for 3 hr at room temperature, and 1:9 for 3 hr at room temperature, then buffer alone, overnight at 4°C). The concentration of BSA-hapten conjugate is determined by spectrophotometric measurement at 280 nm. This conjugate may be stored frozen.

Preparation of Microparticle Reagent

Materials

Carboxylated polystyrene microparticles (0.1 to 0.13 micron, from Seradyn Inc.) N,N-Dimethylformamide (DMF), 1-Hydroxybenzotriazole (NHB.H$_2$O). (NHB stock solution is freshly prepared by dissolving 0.125 g NHB.H$_2$O in 2.5mL DMF, then adding 2.5 mL of deionized water and mixing. The total volume is 5.0 mL at a concentration of 25mg/mL. 1-Cyclohexyl-3-(2-Morpholinoethyl) carbodiimide metho-p-toluene sulfonate (CMC). (CMC stock solution is freshly prepared by disolving 0.25 g in 5 mL $H_2O$ just before use, concentration = 50 mg/mL). Triton X-100, BDZ-BSA conjugate, 0.05M Sodium Bicarbonate pH 8.6, BSA Fraction V Reagent Grade. Microparticle storage buffer: 0.01M Potassium Phosphate buffer pH = 6.0 containing 0.1% $NaN_3$ and 0.1% Triton X-100.

Methods

Ten mL of carboxy modified microparticles (10% solids) are washed with 0.1% Triton X-100 at a ratio of 1:1,000,000 by volume to exchange the detergent. The microparticle concentration is adjusted to 3% by measuring off of a standard curve at 500nm.

Activation: Two mL of NHB stock solution is added slowly and dropwise to 33 mL of rapidly stirring microparticles (3% solids). This microparticle suspension is stirred for 10 minutes at room temperature. Freshly prepared CMC stock solution, 2.9 mL, is added dropwise (ratio of NHB:CMC:microparticle reagent is 3:3:1). After addition of CMC, the microparticle suspension is stirred for 3 hr at room temperature. Activated microparticles are washed with 0.1% Triton X-100 as above to remove the organic solvent and excess activating agents and the concentration is adjusted to 2% solids (measured at 500 nm).

Preparation of Conjugate Solution (ratio of BSA:BSA-BDZ is 1:0.25):

BSA (187.5 mg) and BDZ-BSA (62.5 mg) are dissolved in 50 mL of 0.05 M Sodium Bicarbonate, pH 8.6.
Sensitization: Fifty mL of activated microparticles are quickly added to the rapidly stirring conjugate solution at 25° +/- 1°C. The mixture is incubated 18 hr at same temperature. The microparticles are washed with the storage buffer at 1,000,000 fold to remove the excess BSA. Microparticle reagent is adjusted to 0.7% solids.

Working Microparticle Reagent for the Olympus Analyzer:

Microparticle reagent (0.7%) is diluted to 0.3% with 0.01 M potassium phosphate buffer pH 6.0 containing 0.1% $NaN_3$ and 0.05% Triton X-100.

Preparation of Antibody reagent for test:

Sheep polyclonal antibody selective for Benzodiazepine is diluted appropriately in the following buffer at pH 7.0:
50mM HEPES
0.5%NaCl
0.1% BSA

0.1% $NaN_3$
4% PVP 40 (polyvinylpyrrolidone, average M.W. = 40,000)

Preparation of Diluent for test:

Diluent buffer is at pH 7.0
50mM PIPES
2% NaCl
2.35% PVP 360 (ave. M.W. = 360,000)
0.1% $NaN_3$
0.05% NP40 (Nonidet P-40)

Assay for benzodiazepine:

The diagnostic testing is performed on an Olympus AU 5000 analyzer, which acts as a spectrophotometer. Kinetic interaction of microparticles in suspension yielding microparticle aggregation is measured for turbidimetric changes. Microparticle reagent is used as Reagent 2 (R2), while the Antibody reagent mixed with the Diluent in equal volumes is used as Reagent 1 (R1). The analyzer pipets 15 $\mu$l of the urine sample containing the analyte into a cuvette followed by dispensing 180 $\mu$l of R1. After dispensing 80 $\mu$l of R2 competitive reaction begins. Changes in absorbance are measured for 144 seconds. The absorbance value is compared to a calibration curve, and results are reported in ng/ml. See Figure 3.

The PVP 40, PVP 360 and the antibody concentrations are adjusted to give the best dynamic standard curve with desired performance characteristics around the cut-off, X, 100 ng Nordiazepam/mL.

Example 4

Benzodiazepine Assay - Effect of PVP 40 Concentration

The same materials and methods are used as in Example 3 except that the antibody and diluent reagents are formulated as follows:

Preparation of Antibody reagent for test:

Sheep polyclonal antibody selective for Benzodiazepine is diluted appropriately in the following buffer at pH 7.0:
50mM HEPES
0.5%NaCl
0.1% BSA
0.1% $NaN_3$
PVP 40 at various concentrations
a) 0%
b) 4%
c) 6%

d) 10%

Preparation of Diluent for test:

Diluent buffer is at pH 7.0:
50mM PIPES
2% NaCl
4% PVP 40
0.1% $NaN_3$
0.05% NP40
The results are shown in Figure 4.

**Claims**

1. A microparticle based immunoassay method for the detection of target molecules in a sample of body fluid by agglutinometric determination, the improvement comprising adding to said sample an amount of polyvinylpyrrolidone (PVP) effective to increase the sensitivity and signal of said immunoassay.

2. The method of claim 1 wherein the agglutinometric determination is done by spectrophotometric measurement of turbidity (light absorbance).

3. The method of claim 1 wherein the PVP has an average molecular weight of about 360 kD and is added to a final concentration of at least about 0.6%.

4. A diagnoctic kit for a microparticle based immunoassay for the detection of target molecules in a sample of body fluid by agglutinometric determination comprising a first container containing a reagent comprising antibody selective for said target molecules and a second container containing a reagent comprising microparticles to which are bound immunologically active haptens or antigens selective for said target molecules, the improvement comprising including in either container or the combination of the two an amount of PVP effective to increase the sensitivity and signal of said immunoassay.

5. A diagnostic kit for a microparticle based immunoassay for the detection of target molecules in a sample of body fluid by agglutinometric determination comprising a first container containing a reagent comprising antibody selective for said target molecules, a second container containing a reagent comprising microparticles to which are bound immunologically activated hapten selective for said target molecules, and a third container containing a reagent comprising an aqueous diluent, the improvement comprising including in one of said containers or a combination thereof an amount of PVP effective to increase the sensitivity and signal of said immunoassay.

6. A reagent for a microparticle based immunoassay for the detection of target molecules in a sample of body fluid by agglutinometric determination comprising antibody selective for said target molecules, the improvement comprising including in said reagent an amount of PVP effective to increase the sensitivity and signal of said immunoassay.

7. The reagent of claim 6 wherein the PVP has an average molecular weight of about 360 kD and is present in an amount sufficient to provide a final concentration of at least about 0.6% in said immunoassay.

8. The use of PVP in a microparticle based immunoassay to increase the sensitivity and signal of said immunoassay.

# FIGURE - 1

# FIGURE - 2

# FIGURE - 3

# FIGURE - 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-9 008 321 (DIAGNOSTICA STAGO)<br><br>* Pages 1,2,7,22,23. *<br>--- | 1,2,4-6,8 | G01N33/53<br>G01N33/546 |
| X | FR-A-2 125 000 (F. HOFFMANN-LA ROCHE & CIE.)<br>* Pages 1,2,5,6. *<br>--- | 1-8 | |
| Y,D | US-A-4 480 042 (A.R.CRAIG ET AL.)<br><br>* Abstract; Column 8, line 62-Column 9, line 7; Column 12. *<br>--- | 1,2,4-6,8 | |
| Y | US-A-4 931 385 (E. BLOCK ET AL.)<br><br>* claims *<br>--- | 1,2,4-6,8 | |
| Y | DE-A-3 322 643 (B. EBENER)<br><br>* page 16, line 11 - page 17, line 10; claims *<br>--- | 1,2,4-6,8 | |
| Y | FR-A-2 463 931 (ORION-YHTYMA OY)<br><br>* page 1 - page 3 *<br>--- | 1,2,4-6,8 | **TECHNICAL FIELDS SEARCHED (Int. Cl.5 )** |
| A | CHEMICAL ABSTRACTS, vol. 75, no. 19, 8 November 1971, Columbus, Ohio, US; abstract no. 116534E, S. MATSUZAWA ET AL.: 'Synthetic polymer media for red cell agglutination. Media containing poly(vinylpyrrolidone)(PVP) of two or more different molecular weights.' page 79 ;column 1 ; * abstract * & Nippon Hoigaku Zasshi 1971, 25(1), 1-14.<br><br>----- | 1-3 | G01N |

The present search report has been drawn up for all claims

| Place of search | Date of completion of the search | Examiner |
|---|---|---|
| THE HAGUE | 25 MAY 1992 | HITCHEN C.E. |